# EUROPÄISCHE PATENTSCHRIFT

(11) **EP 0 653 198 B1**
(45) Veröffentlichungstag und Bekanntmachung des Hinweises auf die Patenterteilung: **15.12.2004**
(21) Anmeldenummer: 95100001.7
(22) Anmeldetag: 28.06.1991
(51) Int. Cl.: A61H 39/00, A61N 5/06, H01S 3/10, H01H 27/00

(54) **Sicherheitseinrichtung für elektrische Stromkreise**
Safety switch for electrical circuits
Interrupteur de sécurité pour circuits électriques

(30) Priorität: 29.06.1990 AT 138890; 28.05.1991 AT 107491
(43) Veröffentlichungstag der Anmeldung: 17.05.1995
(62) Teilanmeldung aus: 91890135.6
(73) Patentinhaber: Walter, Helmut Dipl.-Ing.Dr., A-3340 Waidhofen/Ybbs (AT)
(72) Erfinder: Walter, Helmut Dipl.-Ing.Dr., A-3340 Waidhofen/Ybbs (AT)
(74) Vertreter: Casati, Wilhelm, Dipl.-Ing.

(56) Entgegenhaltungen:
- DE-A- 3 228 211
- DE-U- 8 911 606
- US-A- 2 722 575
- US-A- 4 827 385

## Beschreibung

Die Erfindung betrifft eine Sicherheitseinrichtung mit einem Gehäuse für elektrische Stromkreise zur Versorgung von Verbrauchern, mit mindestens einem in den Stromkreis eingebauten Schalter, der von einem elektrisch leitenden Stift od.dgl., gebildet ist, der in das Gehäuse durch einen am Gehäuse angeordneten Schlitz hindurch in das Gehäuse einführbar ist und im eingeführten Zustand zwei im Stromkreis gelegene Kontakte im Gehäuse befindet. Diese Sicherheitseinrichtung eignet sich für Verbraucher, die insbesondere Miniaturlaser sein können. Solche Laser können in der Human- und Veterinärmedizin zur Anwendung kommen, insbesondere für Akupunktur und Wundbehandlung. In dem Gehäuse ist dann die Steuereinrichtung für den Laser angeordnet, wobei auf dem Kopf des Gehäuses verschiedene Objektive auswechselbar anordenbar sind.

Um eine optimale Parallelrichtung des Laserstrahls zu erreichen, wird für solche Laser vorgeschlagen, die Laser-Diode in Richtung der optischen Achse verstellbar im Gehäuse anzuordnen, wodurch es gelingt, die Laserdiode bis auf 1/100 Millimeter genau in den Brennpunkt der Kollimationslinse zu justieren und damit eine optimale Parallelrichtung des Laserstrahls sicherzustellen. Darüberhinaus wird hiedurch die Voraussetzung geschaffen, die Form des Strahlquerschnittes mit Wechselobjektiven beliebig einstellen zu können, weil von einem homogenen, parallelen kreisförmigen Laserstrahl ausgegangen werden kann. Die Randstrahlung des im Querschnitt schlank elliptischen Lichtkegels der Laserdiode kann durch eine Blende ausgeblendet werden, sodaß ein kreisförmiger Strahlquerschnitt überbleibt. Die Blendenwirkung kann durch entsprechende Gestaltung der Weite der Bohrungen im Laserkopf und den Wechselobjektiven realisiert werden. Da ein paralleler Grundstrahl vorliegt, ist die Entfernung eines Wechselobjektives vom Laserkopf unwesentlich, wodurch man in der Objektivberechnung einen Freiheitsgrad erhält. Da der Laserstrahl den Laserkopf bereits gebündelt verläßt, erspart man sich, jedes einzelne der Wechselobjektive mit einer gesonderten Kollimationslinse auszustatten. Dadurch wird einerseits eine Einsparung an Kollimationslinsen erzielt, da nur eine einzige Kollimationslinse erforderlich ist, und anderseits entfällt der sonst bei jedem Wechsel des Objektivs auszuführende Justiervorgang.

Die Geometrie des mit der zuvor beschriebenen Maßnahme erzielbaren parallelen Strahles kann durch nachträgliches Aufschrauben verschiedener Objektive auf den Kopf des Miniaturlasers verändert werden.

Einrichtungen, welche Laserstrahlen mit mehr als 1 mW Ausgangsleistung erzeugen, jedoch auch andere elektrische Stromkreise, welche Verbraucher versorgen und die den menschlichen Körper schädigen können, sind durch mechanische oder elektrische Verriegelungen zu sichern, damit das Gerät von Unbefugten nicht in Betrieb genommen werden kann.

Eine solche Einrichtung ist durch DE-U-8911606 bekannt geworden.

Bekannt ist es, zu diesem Zweck Schlüsselschalter, elektronische Codeschlösser oder Magnetkarten zu verwenden, bei denen der Stromkreis nur durch Personen geschlossen werden kann, die den passenden Schlüssel oder eine passende Magnetkarte besitzen oder die den jeweiligen Code kennen.

Alle diese Sicherheitseinrichtungen haben den Nachteil, daß sie im Hinblick auf ihren Raumbedarf einer Miniaturisierung nicht zugänglich sind oder einen zusätzlichen elektronischen Schaltungsaufwand erforderlich machen, der ebenfalls wieder Platz beansprucht, die Kosten erhöht und einen höheren Stromverbrauch verursacht, der bei Batteriebetrieb besonders nachteilig ist.

Außerdem hindern in einschlägigen Vorschriften enthaltene Zusatzforderungen, wie z.B. daß die Entriegelungsvorrichtung in der "Ein-Stellung" nicht abziehbar sein darf, die Anwendung vieler der am Markt erhältlichen Baugruppen. Aus dem IBM Technical Disclosure Bulletin (Vol.11, No. 10, P. 1311) wurde ein Stöpsel bekannt, der beim Einsetzen in eine Buchse, die zu dem Stöpsel paßt und in welcher sich ein Schalter befindet, eine Feder mechanisch, nach Art eines Nockens anhebt, welche Feder dann den Schalterkontakt schließt, mit dem sie über einen Isolator in Verbindung steht. Der Stöpsel wirkt dabei nicht als leitender Kontaktgeber, somit nicht als leitende Brücke in dem Stromkreis.

Durch die US-PS 2 722 575 wurde ein Sicherheitsschalter für den Zündkreis eines Motorfahrzeuges (Traktors) bekannt. Ein über eine Kette mit dem Unterschenkel des Fahrers verbundener Schaltstift kann durch eine Öffnung in ein Gehäuse eingeschoben werden und schließt im eingeschobenen Zustand zwei elektrisch isoliert am Gehäuse fixierte Kontakte kurz, die im Zündstromkreis des Motorfahrzeuges gelegen sind. Die Öffnung im Gehäuse ist mit Isoliermaterial ausgekleidet, das eine Durchbrechung besitzt, durch welche der Schaltstift in das Gehäuse eingeschoben werden kann. Die beiden Kontakte liegen im Weg des Schaltstiftes.

Durch die US-PS 4 827 385 wurde es bekannt, zum Ein- und Ausschalten einer Taschenlampe, deren Stromquelle eine Stabbatterie ist, einen Taster zu verwenden. Der Taster ist unter Federspannung gegen die Batterie abgestützt. Durch Verschieben des durch eine Feder vorgespannten Tasters wird die Taschenlampe eingeschaltet. Wird der Taster entlastet, kehrt er in seine Ausgangslage zurück, in der der Stromkreis unterbrochen ist.

Aufgabe der Erfindung ist es, eine einfache Lösung des Sicherungsproblems elektrischer Stromkreise für die Versorgung von Verbrauchern anzugeben, insbesondere wenn eine Schädigung von Menschen, durch unbefugte Inbetriebnahme des Verbrauchers auftreten könnte, wie z.B. bei Lasern. Um diese Sicherheitseinrichtung auch für handliche Verbraucher, wie für Miniaturlaser, anwendbar zu machen, schlägt die Erfindung vor, daß das Gehäuse jenes des Verbrauchers selbst ist, daß das Gehäuse als leitendes Element in den Stromkreis einbezogen ist, wobei der an den Stift anliegende Bereich des Schlitzes den einen der durch den Stift zu verbindenden Kontakte bildet, und wobei der zweite der zu verbindenden Kontakte ein Pol der den Verbraucher versorgenden Batterie ist, die mit ihrem anderen Pol über den Verbraucher mit dem Gehäuse leitend in Verbindung steht, wobei der Stift an den einen Pol der Batterie durch das Einschieben in das Gehäuse in Anlage bringbar und in dieser Lage festlegbar ist.

In besonderer, weiterer Ausgestaltung der Erfindung ist die den mit dem Verbraucher verbundenen elektrischen Kontakt bildende Batterie entgegen dem Druck einer Feder im Gehäuse des Verbrauchers verschiebbar gelagert und zur Abstützung der Batterie ein aus elektrisch isolierendem Material bestehender Anschlag im Gehäuse vorgesehen, an dem die Batterie unter dem Druck einer Feder bei aus dem Gehäuse entferntem Stift zur Anlage kommt. Diese Ausgestaltung ist besonders einfach, da die Batterie selbst für das Ein- und Ausschalten benutzt wird.

Der Stift kann auf besonders einfache Weise in der Einschaltstellung gehalten werden, wenn er vom Bart eines Schlüssels gebildet ist, der mit flachen Rippen versehen ist, die durch den Schlitz des Gehäuses hindurchführbar sind und nach Drehung an der Unterseite des den Schlitz aufweisenden Gehäuseteiles abstützbar sind, der insbesondere als Schraubkappe ausgebildet ist.

In besonderer Ausgestaltung der erfindungsgemäßen Einrichtung ist der Anschlag aus isolierendem Material als Ring ausgebildet und in den, den Schlitz aufweisenden Gehäuseteil eingesetzt. Durch diese Ausgestaltung wird ein sicherer Sitz der Batterie in der abgeschalteten Stellung erzielt, da die Abstützung an der Stirnfläche der Batterie erfolgt.

Die Erfindung wird nachstehend anhand der Zeichnung, die beispielshaft erfindungswesentliche Details veranschaulicht, näher erläutert. Es zeigen,
Fig. 1 in einem teilweisen Längsschnitt einen Miniaturlaser,
Fig. 2 einen Teil eines Miniaturlasers gemäß Fig. 1 in einem Längsschnitt, mit einer erfindungsgemäßen Sicherheitseinrichtung gegen unbefugte Inbetriebnahme,
Fig. 3 eine Ansicht auf den in Fig. 2 dargestellten Teil des Miniaturlasers in Richtung des Pfeiles III in Fig. 2,
Fig. 4 einen der Fig. 2 entsprechenden Schnitt, jedoch in Einschaltstellung, in einem Schnitt gemäß Linie IV-IV in
Fig. 5, die eine Ansicht in Richtung des Pfeiles V auf die Fig. 4 wiedergibt,
Fig. 6 einen Schnitt entlang der Linie VI-VI in Fig. 5, und
Fig. 7 schematisch den grundsätzlichen Aufbau einer Sicherheitseinrichtung gegen unbefugte Inbetriebnahme des Miniaturlasers.

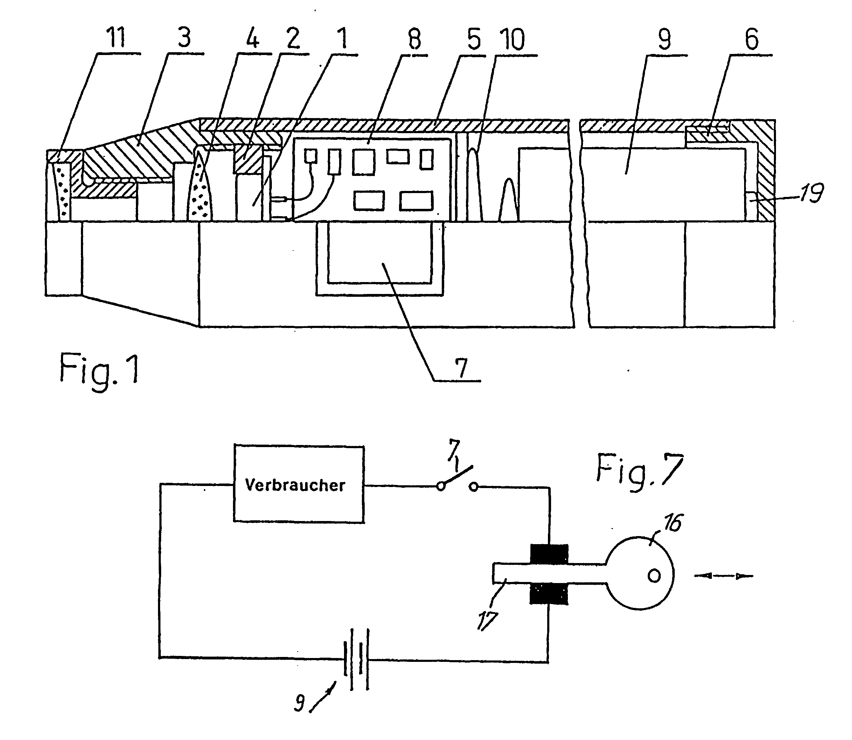

Die Laserdiode 1 ist in eine Gewindehülse 3 befestigt, insbesondere eingeklebt und kann in Axialrichtung der Gewindehülse 3, die den konischen Vorderteil des Gehäuses 5, 6 bildet, genau in den Brennpunkt der Sammellinse (Kollimationslinse) 4 gedreht werden, wo sie dann, bevorzugt durch einen Klebertropfen fixiert wird. Die Gewindehülse 3 des Miniaturlasers ist in den als Rohr 5 ausgebildeten Teil des Gehäuses eingepreßt, das die notwendige elektrische Verbindung zu einer Verschlußkappe 6 des Gehäuses herstellt. Mit 7 ist in Fig. 1 ein Ein-Austaster bezeichnet. Unterhalb des Ein/Aus-Tasters ist die in SMD-Technik gefertigte Regelschaltung 8 für die Laserdiode 1 angeordnet, die von Batterien 9 versorgt wird. Um einen guten elektrischen Kontakt zwischen den Batterien 9 und dem Gehäuse 5, 6 zu erzielen, ist eine Andruckfeder 10 vorgesehen, welche die Batterien 9 gegen die elektrisch leitende Verschlußkappe 6 drückt. Da alle Teile innerhalb der den konischen Vorderteil bildenden Hülse 3 aus einem leitfähigen Material angefertigt sind, ist durch diese Konstruktion gewährleistet, daß zum einen die Laserdiode 1 über das ganze Gehäuse 5, 6 gekühlt wird und zum anderen ein Minimum an Versorgungsleitungen für die Regelschaltung 8 erforderlich ist. Auf die Hülse 3, die den Kopf des Gehäuses bildet, werden Wechselobjektive 11 aufgeschraubt, die als Drehteile ausgeführt sind.

Die Kollimationsoptik 4 dient dazu, die divergente Strahlung der Laserdiode 1 in Richtung der optischen Achse des Lasers zu bündeln. Die Wechselobjektive 11 bedürfen demgemäß keiner weiteren Kollimationslinse.

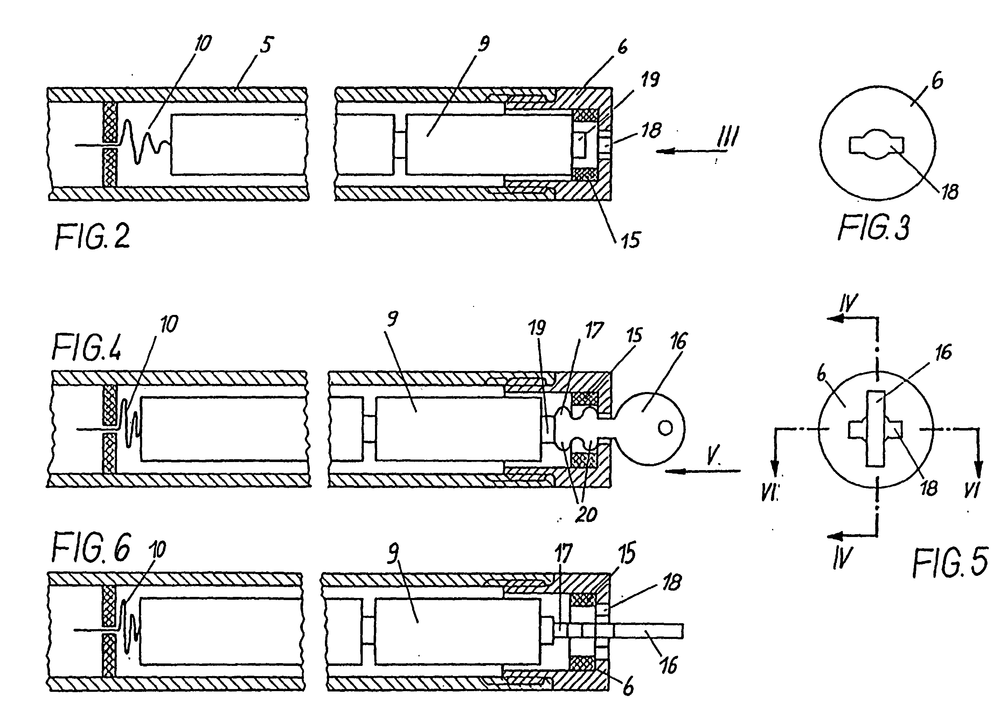

Bei der in den Fig. 2 - 7 dargestellten Sicherheitseinrichtung gegen unbefugtes Inbetriebnehmen des Miniaturlasers ist in den Stromkreis zur Versorgung des Verbrauchers (Lasers) mindestens ein Schalter eingebaut. Dieser ist von einem elektrisch leitenden Stift 17 gebildet, der durch einen Schlitz 18 in einem elektrisch leitenden, den Verbraucher aufweisenden Gehäuse 5, 6 einführbar ist, wobei im Weg des Stiftes 17 ein mit dem Verbraucher elektrisch verbundener Kontakt 19 gelegen ist. Dieser Kontakt ist von einem Pol einer, entgegen dem Druck einer Feder 10 im Gehäuse 5, 6 verschiebbar gelagerten Batterie 9 gebildet, für deren Abstützung ein aus elektrisch isolierendem Material bestehender Anschlag 15 vorgesehen ist, an dem die Batterie 9 unter dem Druck einer Feder 10 bei aus dem Gehäuse 5, 6 entferntem Stift 17 zur Anlage kommt. Der den Schalter bildende Stift 17 kann vom Bart eines Schlüssels 16 gebildet sein, der mit flachen Rippen 20 versehen ist, die durch den Schlitz 18 des Gehäuses 5, 6 hindurchführbar sind und nach Drehung, an der Unterseite des den Schlitz 18 aufweisenden Gehäuseteiles 6, der insbesondere als Schraubkappe ausgebildet ist, abstützbar sind. Der Anschlag aus isolierendem Material, gegen den bei entferntem Stift 17 die Batterien 9 unter dem Druck der Andruckfeder 10 anliegen, kann als Ring ausgebildet sein. Der Ring ist bei dem dargestellten Ausführungsbeispiel in den, den Schlitz 18 aufweisenden Gehäuseteil (Verschlußkappe 6) eingesetzt und so dick ausgeführt, daß in der Aus-Stellung (Fig. 2) der der Andruckfeder 10 abgekehrte Pol (Kontakt 19) der äußeren der Batterien 9, die Metallfläche der Verschlußkappe 6 nicht berühren kann. Wird nun - ausgehend von dieser Stellung - der zum Schlüssel 16 gestaltete Stift 17 in den Schlitz 18 der Verschlußkappe 6 eingeführt, so drückt dieser Stift 17 auf den der Federseite abgewandten Pol der äußeren der Batterien 9 und stellt damit einen elektrischen Kontakt zu den Batterien her. Wird der Schlüssel 16 um 90° gedreht und anschließend an die Drehung losgelassen, so werden aufgrund der federnden Vorspannung der Batterien 9, die Rippen 20 des Schlüssels 16 gegen die leitfahige Rückwand der Abdeckkappe 6 gedrückt und ein elektrischer Kontakt zwischen den Batterien 9 und der Abdeckkappe 6, bzw. dem Gehäuse 5, 6, hergestellt. Der Schlüssel 16 kann nun, ohne den Kontakt wieder zu unterbrechen, nicht mehr abgezogen werden. Dieser Zustand entspricht der "Ein-Stellung" (Fig. 4 - 7). Wird der Schüssel 16 erneut um 90° gedreht, wird dieser von den federnd vorgespannten Batterien 9 ausgestoßen, die Batterien werden gegen den Anschlag 15 der Verschlußkappe 6 gepreßt, und die Verbindung zum Gehäuse 5, 6 unterbrochen (Fig. 2).

## Patentansprüche

1. Laser, dessen Laserdiode (1) gemeinsam mit einer die Laserdiode (1) versorgenden Batterie (9) in einem Gehäuse (5, 6) angeordnet ist, das mit einem Ein-/Austaster zum Öffnen und Schließen des von der Batterie (9) versorgten Stromkreises und mit einer ebenfalls im Gehäuse (5, 6) des Lasers angeordneten Sicherheitseinrichtung versehen ist, **dadurch gekennzeichnet, daß** die Sicherheitseinrichtung als Schalter in Form eines elektrisch leitenden Stiftes (17) ausgebildet ist, der in das Gehäuse (5, 6) des Lasers durch einen am Gehäuse (5, 6) angeordneten Schlitz hindurch einführbar ist und im eingeführten Zustand zwei im Stromkreis der Batterie (9) gelegene Kontakte verbindet, die im Gehäuse (5, 6) angeordnet sind, das als leitendes Element in den Stromkreis einbezogen ist, wobei der eine der durch den elektrisch leitenden Stift (17) zu verbindenden Kontakte der an dem Stift (17) anliegende Bereich des Schlitzes (18) ist und der zweite der durch den Stift (17) zu verbindenden Kontakte ein Pol (19) der die Laserdiode (1) versorgenden, entgegen dem Druck einer Feder (10) im Gehäuse (5, 6) verschiebbar gelagerten und am eingeschobenen Stift (17) abgestützten Batterie (9) ist, die mit ihrem anderen Pol über die Laserdiode (1) mit dem Gehäuse (5, 6) leitend in Verbindung steht, wobei der Stift (17) im eingeführten Zustand am erstgenannten Pol (19) der Batterie (9) anliegt und in dieser Lage dadurch festgelegt ist, daß der Stift (17) vom Bart eines Schüssels (16) gebildet ist, der mit flachen Rippen (20) versehen ist, die durch den Schlitz (18) des Gehäuses (5, 6) hindurchführbar sind und nach Drehung an der Unterseite des den Schlitz (18) aufweisenden Gehäuseteiles (6), der insbesondere als Schraubkappe ausgebildet ist, abgestützt sind.

2. Laser nach Anspruch 1, **dadurch gekennzeichnet, daß** bei aus dem Gehäuse (5, 6) entferntem Stift (17) die Batterie (9) unter dem Druck der Feder (10) an einem aus elektrisch isolierendem Material bestehenden Anschlag (15) abgestützt ist.

3. Laser nach Anspruch 2, **dadurch gekennzeichnet, daß** der Anschlag (15) aus isolierendem Material als Ring ausgebildet ist und in den, den Schlitz (18) aufweisenden Gehäuseteil (6) eingesetzt ist.

## Claims

1. Laser, of which the laser diode (1) is arranged together with a battery (9) supplying the laser diode (1) in a housing (5, 6), which is provided with an on/off switch for opening and closing the power circuit supplied by the battery (9), and with a safety device likewise arranged in the housing (5,6) of the laser, **characterised in that** the safety device is designed as a switch in the form of an electrically-conductive pin (17), which can be introduced into the housing (5, 6) of the laser through a slot arranged on the housing (5,6), and in the introduced state connects two contacts located in the circuit of the battery (9), which are arranged in the housing (5,6), which is incorporated into the circuit as a conductive element, whereby one of the contacts which is to be connected by the electrically conductive pin (17) is the area in contact with the pin (17), and the second of the contacts which is to be connected by the pin (17) is a pole (19) of the battery (9), which supplies the laser diode (1), and which is supported at the pin (17) which has been pushed in, and is mounted in a manner such as to be displaceable in the housing (5,6) against the pressure of a spring (10), said battery being in conductive contact with its other pole with the housing (5, 6) via the laser diode (1), whereby the pin (17) in the inserted state is in contact with the pole (19)first referred to of the battery (9), and in this position is secured **in that** the pin (17) is formed from the flag of a key (16), which is provided with flat ribs (20), which can be introduced through the slot (18) in the housing (5,6), and, after rotation, are supported on the under side of the housing part (6) exhibiting the slot (18), said housing part being designed in particular as a screw cap.

2. Laser according to claim 1, **characterised in that**, when the pin (17) is removed from the housing (5, 6), the battery (9) is supported at a stop element (15), consisting of electrically insulating material, under the pressure of the spring (10).

3. Laser according to claim 2, **characterised in that** the stop element (15) is formed from insulating material as a ring, and is inserted into the part of the housing (6) exhibiting the slot (18).

## Revendications

1. Laser dont la diode laser (1) est disposée dans un boîtier (5, 6) conjointement avec une batterie (9) alimentant la diode laser (1), ledit boîtier étant muni d'un commutateur marche/arrêt pour ouvrir et fermer le circuit alimenté par la batterie (9) et d'un dispositif de sécurité disposé également dans le boîtier (5, 6) du laser, **caractérisé en ce que** le dispositif de sécurité est réalisé sous forme d'interrupteur sous forme d'une broche conductrice électrique (17), qui est insérable dans le boîtier (5, 6) du laser à travers une fente disposée sur le boîtier (5, 6). et qui relie à l'état inséré deux contacts placés dans le circuit de la batterie (9), lesdits contacts étant disposés dans le boîtier (5, 6), qui est inséré dans le circuit en tant qu'élément conducteur, où le premier des contacts à relier par la broche conductrice électrique (17) est la zone de la fente (18) adjacente à la broche (17) et le deuxième des contacts à relier par la broche (17) est un pôle (19) de la batterie (9) alimentant la diode laser (1), logée dans le boîtier (5,6) de manière coulissante à l'encontre de la pression d'un ressort (10) et appuyée contre la broche (17) intercalée, ladite batterie étant en contact conducteur avec le boîtier (5, 6) avec son autre pôle sur la diode laser (1), la broche (17) reposant à l'état inséré sur le premier pôle (19) mentionné de la batterie (9) et étant fixée dans cette position du fait que la broche (17) est formée par la barbe d'une clé (16) qui est munie de nervures plates (20) qui peuvent être passées à travers la fente (18) du boîtier (5, 6) et qui sont appuyées, après rotation, contre la face inférieure de la partie de boîtier (6) présentant la fente (18), la partie de boîtier (6) est réalisée en particulier sous forme de couvercle fileté.

2. Laser selon la revendication 1, **caractérisé en ce qu'**en cas de broche (17) retirée du boîtier (5, 6), la batterie (9), sous la pression du ressort (10), est appuyée contre une butée (15) composée d'un matériau isolant électrique.

3. Laser selon la revendication 2, **caractérisé en ce que** la butée (15) en matériau isolant est réalisée sous forme d'anneau et est insérée dans la partie de boîtier (6) présentant la fente (18).
